(19) **Europäisches Patentamt / European Patent Office / Office européen des brevets**

(11) **EP 4 278 364 B1**

(12) **EUROPEAN PATENT SPECIFICATION**

(45) Date of publication and mention
of the grant of the patent:
**05.08.2026 Bulletin 2026/32**

(21) Application number: **22701136.8**

(22) Date of filing: **13.01.2022**

(51) International Patent Classification (IPC):
***G16H 50/20*** *(2018.01)*

(52) Cooperative Patent Classification (CPC):
**G16H 50/20**

(86) International application number:
**PCT/US2022/012303**

(87) International publication number:
**WO 2022/155328 (21.07.2022 Gazette 2022/29)**

(54) **SENSING OF BIOLOGICAL CELLS IN A SAMPLE FOR CELL TYPE IDENTIFICATION**

ERFASSUNG BIOLOGISCHER ZELLEN IN EINER PROBE ZUR ZELLTYPIDENTIFIZIERUNG

DÉTECTION DE CELLULES BIOLOGIQUES DANS UN ÉCHANTILLON POUR IDENTIFIER UN TYPE DE CELLULE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.01.2021 US 202163137843 P**
**17.03.2021 EP 21315038**

(43) Date of publication of application:
**22.11.2023 Bulletin 2023/47**

(73) Proprietor: **Sanofi**
**75017 Paris (FR)**

(72) Inventors:
• **DE RINALDIS, Emanuele**
**Bridgwater, New Jersey 08807 (US)**
• **SAVOVA, Virginia**
**Bridgewater, New Jersey 08807 (US)**

• **CHAMBERLAIN, Mathew**
**Bridgewater, New Jersey 08807 (US)**

(74) Representative: **McDougall, James et al**
**Venner Shipley LLP**
**TIDE Bankside**
**8 Emerson Street**
**London SE1 9DU (GB)**

(56) References cited:
**WO-A1-00/49391        JP-A- 2020 153 946**
**US-A1- 2020 090 782    US-A1- 2020 176 080**

• **QIU WEI ET AL: "Multi-label Detection and Classification of Red Blood Cells in Microscopic Images", 2020 IEEE INTERNATIONAL CONFERENCE ON BIG DATA (BIG DATA), IEEE, 10 December 2020 (2020-12-10), pages 4257 - 4263, XP033889596, DOI: 10.1109/ BIGDATA50022.2020.9377782**

**Description**

**TECHNICAL FIELD**

This document describes technology that uses sensor data to identify and classify biological cells.

**BACKGROUND**

Single-cell analysis in cellular biology is the study of genomics, transcriptomics, proteomics, metabolomics and cell-cell interactions at the single cell level. Due to the heterogeneity seen in both eukaryotic and prokaryotic cell populations, analyzing a single cell makes it possible to discover mechanisms not seen when studying a bulk population of cells. Technologies such as fluorescenceactivated cell sorting (FACS) allow the precise isolation of selected single cells from complex samples, while high throughput single cell partitioning technologies enable the simultaneous molecular analysis of hundreds or thousands of single unsorted cells.

**SUMMARY**

Technology that identifies single cells, including of previouslyunknown cells, is described. Sensor data is collected from a sample of biological cells, and machine-learning classifiers can be used to classify each cell sensed in the sample. In order to train these classifiers, training sets are created based on cell identification. Some cells in samples are relatively populous, and thus may be used directly as a training corpus. However, rare cells may not provide enough data points, and may not provide data points with enough discriminatory power, to train reliable machine-learning classifiers. For these rare cells, a corpus may be bootstrapped based on the rare examples combined with mathematical noise that has statistical profiles that match known variations in known cells. In this way, high-quality data sets may be generated, and from these high-quality data sets, high-quality classifiers may be trained. By using the high-quality classifiers, cell samplers and their associated computing apparatus can better sense and identify biological cells.

In an example, a system may be used for the sensing of data from a sample of biological cells. The system includes a cell sampler comprising a sample receiver and one or more sensors; wherein the cell sampler is configured to sense, with the sensors, physical phenomena of biological cells in the sample receiver; and transmit, to processing apparatus, sensor-data generated from the sensing of the biological cells. The system includes processing apparatus comprising computer memory and one or more processors, the processing apparatus configured to: receive, from the cell sampler, the sensor-data; identify, using the sensor-data, individual cells of the biological cells; for each individual cell: generate, using the sensor-data, a cell type for the individual cell; generate, using the sensor-data, a feature vector for the individual cell; classify, using the sensor-data, at least some of the cell types as uncommon; for each uncommon cell type: access the feature vectors of individual cells of the uncommon cell type; generate bootstrap vectors for the uncommon cell type by applying noise to the feature vectors of individual cells of the uncommon cell type; and generate a cell-corpus by aggregating the bootstrap vectors and the feature vectors of individual cells of the common cell type. Other examples include methods, computer-readable media, devices, and software.

Examples can include some, all, or none of the following features. The processing apparatus is further configured to perform at least one of the group consisting of i) storing at least one of the cell-corpuses to a data repository as a result of sensing of the biological cells; ii) transmitting a report of at least one of the cell-corpuses across a data network, and iii) initiating, in response to generating at least one of the cell-corpuses, an automated process without specific user-input to initiate the automated process. To generate, using the sensor-data, a cell type for the individual cell, the processing apparatus is further configured to submit the sensor-data to one or more machine-learning classifiers configured to receive, as input, the sensor-data and generate, as output, an indication of cell type. The one or more machine-learning classifiers include a plurality of classifiers arranged in a hierarchical decision-tree that, at each of a plurality of nodes of the decision-tree having an ensemble of machine-learning classifiers that are configured to vote on a classification. A root node of the decision-tree has a child for immune cells and a child for non-immune cells. The machine-learning classifier was trained on an initial-corpus of training data; and the processing apparatus is further configured to: generate an updated-corpus of training data by incorporating at least one of the cell-corpuses to the initial-corpus; and training updated machine-learning classifiers with the updated corpus. The processing apparatus is further configured to: identify one of the individual cells as a high-entropy cell due to the high-entropy cell being found in a cluster with a high level of entropy; disassociate, from the high-entropy cell, the generated cell type; and classify the high-entropy cell as a novel cell type. The processing apparatus is further configured to: identify one of the individual cells as a high-entropy cell due to the high-entropy cell being found in a cluster with a high level of entropy; disassociate, from the high-entropy cell, the generated cell type; and perform at least one of the group consisting of i) storing information about the high-entropy cell to a data repository as a result of sensing of the biological cells; ii) transmitting a report about the high-entropy cell across a data network, and iii) initiating, in response to identifying the high-entropy cell, an automated process without specific user-input to initiate the automated process. Identifying one of the individual cells as a high-entropy cell comprises calculating a Shannon-entropy value for the high-entropy cell. The noise is generated based on statistical measures of previously-analyzed cells. The processing apparatus is further configured to generate the noise based on statistical measures of the sensor-data.

Implementations can include any, all, or none of the following features. The technology of single cell analysis is advanced. Machine-learning classifiers can be trained on data about cells that are so rare, training data would not be otherwise available without this technology. This allows for the creation of sensors and their associated controllers that can classify those rare cells when encountered. Further, previously unknown cell types can be identified and analyzed. This analysis can be incorporated into the classifiers, improving their performance when encountering uncommon cells a second time.

Other features, aspects and potential advantages will be apparent from the accompanying description and figures.

## DESCRIPTION OF DRAWINGS

FIG. 1 shows an example system that senses data from a sample of biological cells.
FIG. 2 shows an example of data that can be used in the sensing of data from a sample of biological cells.
FIG. 3 shows a swimlane diagram of an example process of sensing data from a sample of biological cells.
FIG. 4 shows a schematic diagram of an example of a computing device and a mobile computing device.
FIG. 5 shows an example process of sensing data from a sample of biological cells.

Like reference symbols in the various drawings indicate like elements

## DETAILED DESCRIPTION

Cell identification is improved by the use of sensing and classification technologies, which begins with training data from common cell types, updates the training data by bootstrapping data for rare cells, and then trains machine-learning classifiers from the training data. These classifiers can then be arranged in a hierarchical decision tree that can be used to classify sensed cells.

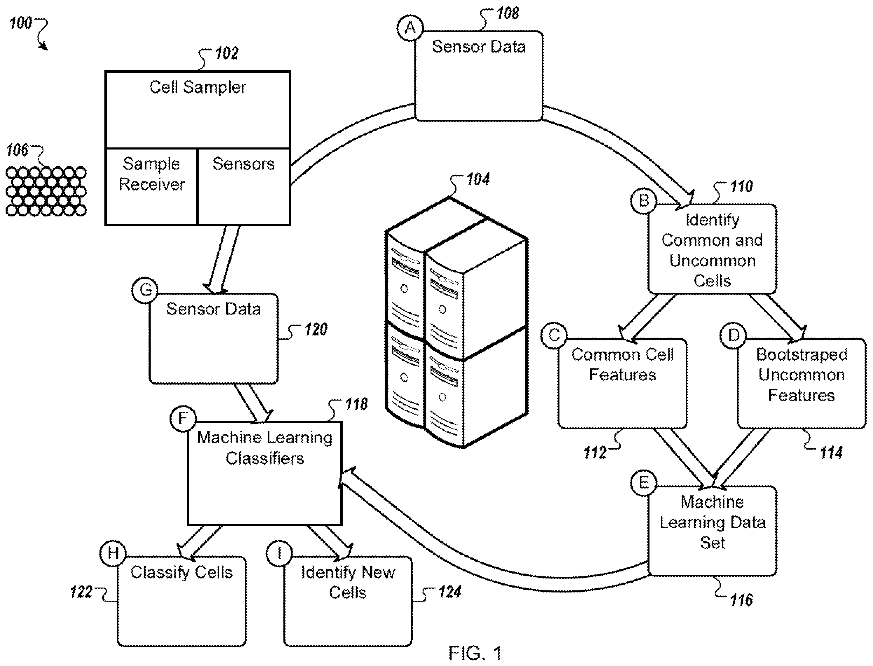

FIG. 1 shows an example system 100 that senses data from a sample of biological cells. In the system 100 a cell sampler 102 works in conjunction with processing apparatus 104 to generate machine-learning classifiers 118 that can be used to classify new cells and identify new cell types not previously known.

The cell sampler 102 is any one or combinations of devices that is capable of receiving samples of cells 106 in a sample receiver and sensing, with one or more sensors, physical phenomena of the cells 106. Example cell samplers 102 include, but are not limited to well-based or droplet-based cell sequencers. Some example cell samplers 102 include devices that use microfluidic structures to perform single-cell partitioning and barcoding. In some examples, the cell sampler 102 performs sensings that are multidimensional and transcriptomic.

The cell sampler 102 is in data communication with processing apparatus 104, which includes computer memory and one or more processors that are capable of performing instructions to receive data, perform data computations, generate reports, transmit data over networks, etc. As will be appreciated, the processing apparatus 104 may include one or more devices such as computers, monitors, data networking equipment, etc. Some or all of the apparatus 104 may be physically integrated with the cell sampler 102, for example, in the form of a dedicated device controller. Some or all of the apparatus 104 may be geographically remote but in data communication over one or more networks including the Internet.

The system 100 can operate to create training data on which machine-learning classifiers 118 can be trained. The cell sampler 102 receives a sample of cells 106 and senses physical phenomena of the cells 106. From this sensing, sensor data 108 is generated, recording data reflecting the phenomena. Individual cells 106 are identified - that is to say, many different single cells 106 are identified - and classified as common or uncommon 110. For the cells 106 of the common cell type, common cell features 112 are identified and associated with their corresponding type. For the cells 106 of the uncommon type, extra features 114 are bootstrapped from the features that are directly sensed and recorded in the sensor data 108.

The common cell features 112 and the bootstrapped features 114 are combined into one or more machine-learning data set 116. By using the bootstrapped features 114, the apparatus 104 is able to construct a data set suitable for the training of classifiers, even in the face of cell types for which only one or a few cells are available. Such technology can advantageously train machine-learning classifiers by sensing fewer physical phenomenon than would otherwise be possible. This can have the advantageous feature of being able classifying more uncommon cell types than would otherwise be possible.

With the one or more machine-learning classifiers 118, further sensor data 120 can be submitted to the classifiers 118 for analysis. For cell types already seen, include those uncommon cells that would otherwise be too rare to allow for machine-learning training, the sensor data 120 can be classified into cell classifications 122. Furthermore, new cells types can be identified 124 for recordation and/or study. This can advantageously advance the technology of single-cell identification, classification, and sequencing.

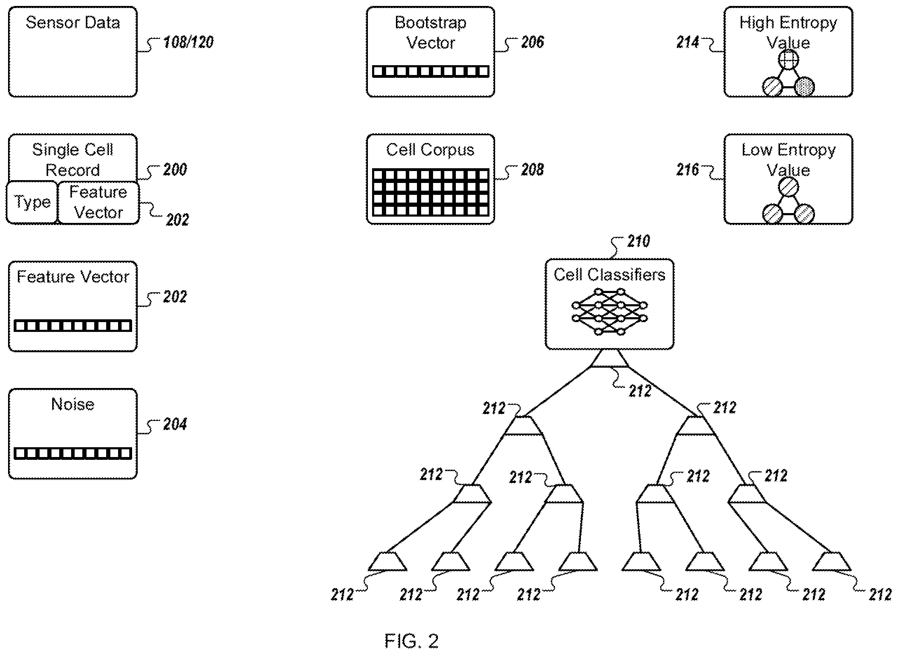

FIG. 2 shows an example of data that can be used in the sensing of data from a sample of biological cells. For example, the data shown in FIG. 2 may be used by the system 100 or other systems. The data shown here may be recorded in one or more data store, used in short-term memory storage by processors, transmitted over data networks, etc.

Sensor data 108/120 includes data generated by sensors and/or controllers that operate those sensors. Various types of sensors include various types of hardware that, under some environmental conditions, generate electrical signals differentially based on a feature of the environment. Said a different way, the sensor data 108/120 reflects a physical condition of the cells 106.

A single cell record 200 records information about a particular cell in a sample of cells. The record 200 may be in a structured format with fields to store, for example, a designation of cell type, a feature vector 202, a date created, a sampleidentifier from which the single cell is a member, etc., and/or references to similar fields of data.

The feature vector 202 may store a collection (e.g., an array, a list, a vector) of features that were determined for a single cell and may be stored as part of the single cell record 200. In one implementation, each index of the feature vector 202 records a value to reflect a single gene expression of the single cell, but other schemes of data storage may be used.

Noise 204 may store a collection (e.g., an array, a list, a vector) of random or pseudo-random values that have been adjusted to conform to one or more statistical rules. For example, a set mean, standard deviation, and range values may be compiled based on records of variation of known-common cells. The noise 204 may also exhibit the same mean, standard deviation, and range values. In some cases, the noise 204 is generated based on statistical measures of previously-analyzed cells in the system 100. For example, the processing apparatus 104 may be configured to generate the noise 204 based on statistical measures of the sensor-data.

A bootstrap vector 206 may store a collection (e.g., an array, a list, a vector) of features that are generated by applying the noise 204 to a feature vector 202. In such a case, the bootstrap vector 206 can, for example, values that are similar to, and within reasonable variation from, the feature vector 202 of an uncommon-type cell. This may be advantageous, for example, in situations where uncommon cells are not found in great-enough abundance to produce feature vectors 202 for a particular task. One such task is the training of machine-learning classifiers, though others are possible.

A cell corpus 208 contains data representative of many cells. For example, the cell corpus 208 can include feature vectors 202 as well as bootstrap vectors 206. The cell corpus 208 can be used for a number of useful tasks. One such task is the training of machine-learning classifiers, though others are possible.

Cell classifiers 210 include functions that are configured to receive, as input, a feature-vector 202 and return, as output, a classification value. For example, an unclassified, recently sensed cell's feature vector 202 may be submitted to the cell classifiers 210 for classification for the first time.

The cell classifiers 210 may be arranged in a hierarchical decision-tree that, at each of a plurality of nodes 212 of the decision-tree having an ensemble of machine-learning classifiers 210 that are configured to vote on a classification. As such, the cell classifiers 210 can provide a single classification, a series of classifications with confidence values, and classifications at various levels of specificity corresponding to the various levels of the decision tree.

Entropy values 214 and 216 can record, for an individual cell or cluster of cells, an entropy value. For high-entropy clusters or cells in high-entropy clusters, a high value 214 can be recorded. For low-entropy clusters or cells in low-entropy clusters, a low value 216 may be recorded.

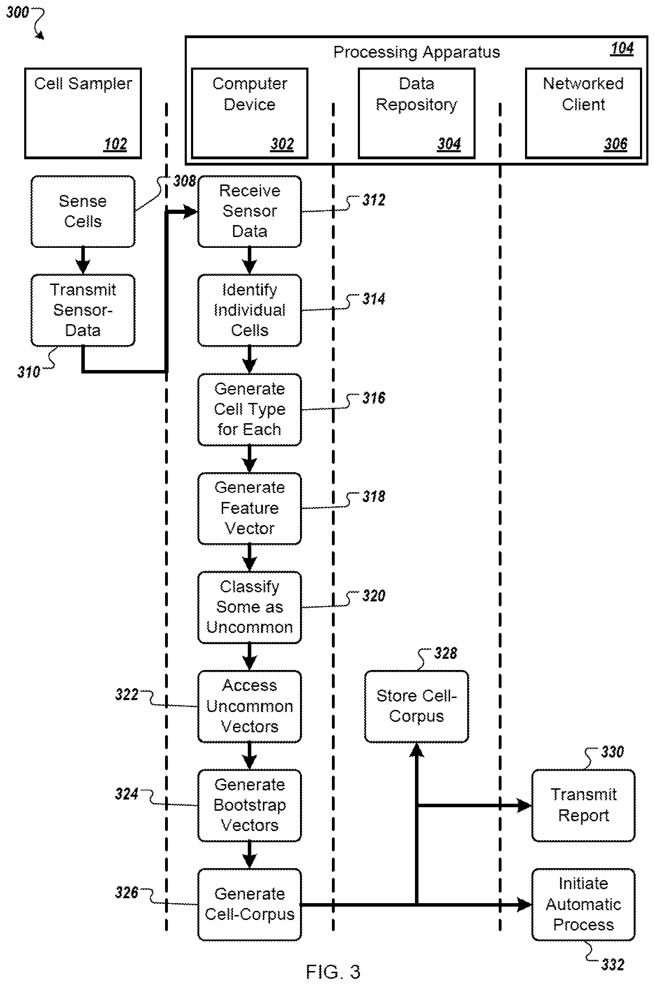

FIG. 3 shows a swimlane diagram of an example process of sensing data from a sample of biological cells. The process 300 can be performed by, for example, the system 100, and as such elements of the system 100 will be used for this example, however other systems may be used to perform the process 300 and other processes.

In this example, the processing apparatus 104 includes a computer device 302, a data repository 304, and a networked client 306. The devices 302-306 are each geographically separated and connected with one or more data networks, including the Internet. However, other elements of the processing apparatus 104 can be used in other examples.

The cell sampler 102 is configured to sense 102, with the sensors, physical phenomena of biological cells in the sample receiver. For example, a handler (e.g., a human technician or an automated material-handling robot) can load a sample of biological cells into the sample receiver of the cell sampler 102 and may issue a command (e.g., pressing a button or transmitting a data message) to analyze the cells.

The cell sampler 102 is configured to transmit 310, to processing apparatus, sensor-data generated from the sensing of the biological cells and the processing apparatus 104 is configured to receive 312, from the cell sampler 102, the sensor-data. For example, the cell sampler 102 can send a data message from the sensing directly to the client device 302, may store the message in the data repository 304 and send a message with a pointer to the data to the computer device 302, or otherwise communicate the data.

The processing apparatus 104 is configured to identify 314, using the sensor-data, individual cells of the biological cells. For example, the computer device 302 may parse the received data and create, for each single cell, a corresponding unique identifier (e.g., a barcode).

For each individual cell, the processing apparatus 104 is configured to generate 316, using the sensor-data, a cell type for the individual cell. For example, the computer device 302 may use one or more techniques to classify each single cell. The computer device 302 can submit the sensor-data to one or more machine-learning classifiers configured to receive, as input, the sensor-data and generate, as output, an indication of cell type. This can produce an indication of cell type for each unique identifier and thus single cell.

In some cases, the one or more machine-learning classifiers include a plurality of classifiers. These classifiers can work together (e.g., by pooling votes or confidence levels) to create the classification. These classifiers may be arranged in a hierarchical decision-tree. This tree can, at each of the nodes of the have an ensemble of machine-learning classifiers that are configured to vote on a classification. This voting can be used to create the classification.

The tree can be general purpose, and thus used when cells of completely unknown type are to be received or otherwise. In some cases, the tree may be structured for particular uses. One such use is the discrimination and classification of immune cells. In such a case, the tree may be organized so that a root node of the decision-tree has a child for immune cells and a child for non-immune cells. Thus, each cell may be first classified as immune (e.g., and kept for further analysis) or non-immune (e.g., discarded from further analysis). Further classification can occur of the immune cells, the non-immune cells, or both the immune cells and the non-immune cells.

For each individual cell, the processing apparatus 102 is configured to generate 318, using the sensor-data, a feature vector for the individual cell. This vector can record various features of the cell. As will be appreciated, each cell may have a corresponding vector of the same format, with the first element of the vector being used for the same data across all vectors, the second element of the vector being used for another same data across all vectors, etc.

In addition to the uses described here, the feature vector may be used as an input to other operations. For example, the feature vector may be used in deconvolution and signature analysis, as well as other purposes.

For each individual cell, the processing apparatus 102 is configured to classify 320, using the sensor-data, at least some of the cell types as uncommon. For example, any cell type with less than a threshold number of cells in the sample may be classified as uncommon. This threshold may be a static value (e.g., 2, 10, 100) or a derivative value of another value (e.g., less than two standard deviations from the mean, the Nleast-populous cell types). This other value may be a value related to the sample (i.e. for finding uncommon cells in the sample) or of another data set (i.e. for finding uncommon cells when considering all known cells available).

For each uncommon cell type, the processing apparatus 104 is configured to access 322 the feature vectors of individual cells of the uncommon cell type. For example, the computer device 302 can access all feature vectors and filter out the feature vectors of the common cells. In another example, the computer deice 302 can construct and submit a query that returns only feature vectors of uncommon cells.

For each uncommon cell type, the processing apparatus 102 is configured to generate 324 boostrap vectors for the uncommon cell type by applying noise to the feature vectors of individual cells of the uncommon cell type. For example, each feature vector here may have $I$ elements with data than can range from $0..M$. The noise may contain values of $0..M$ that are random and conform to variations found among common cell types. The computer device 302 can combine each feature vector element with the next unused number in the noise with wraparound addition so that the value remains between $0...M$ but is altered by the noise. In addition to wraparound addition, other forms of combination may be used. This may depend on, for example, the way that the data is represented and stored.

The processing apparatus 102 is configured to generate 326 a cell-corpus by aggregating the bootstrap vectors and the feature vectors of individual cells of the common cell type. For example, the computer device 302 can begin with all feature vectors generated, or only the feature vectors of the common cells, and add to that collection all of the bootstrap vectors 324. In some cases, the computer device 302 may be configured to run one or more post-processing tests on the corpus to ensure that it meets minimum standards established for a particular use. For example, a minimum number of data entries for a machine-learning classification may be established.

The processing apparatus 102 is further configured to store 328 at least one of the cell-corpuses to a data repository as a result of sensing of the biological cells. For example, the data repository may store, in long-term and stable storage, the cell-corpus. The data repository 304 may then respond to queries on the cell-corpus when the data repository 304 receives such queries.

The processing apparatus 102 is further configured to transmit 330 a report of at least one of the cell-corpuses across a data network. For example, the networked client can send, to a clinician, a report about a patient's cells for use in the diagnostic care of the patient.

The processing apparatus 102 is further configured to initiate 332, in response to generating at least one of the cell-corpuses, an automated process without specific user-input to initiate the automated process. For example, the networked client 306 can run one or more quality checks on the corpus and, if the corpus passes these checks, initiate one or more processes.

On example of such processes is the training of machine-learning classifiers. In some cases, the classifiers used by the computer device 302 may have been created in this way. That is, the machine-learning classifier were trained on an initial-corpus of training data, which was later updated. In such a case, the processing apparatus 102 is configured to generate an updated-corpus of training data by incorporating at least one of the cell-corpuses to the initial-corpus. The processing apparatus 104 in such a case is configured to train updated machine-learning classifiers with the updated corpus. As such, this updated corpus would include a greater number of cell types, allowing for more flexible classification.

One example of such processes is the categorization of high-entropy cells. For example, the processing apparatus can identify one of the individual cells as a high-entropy cell due to the high-entropy cell being found in a cluster with a high level of entropy, including but not limited to Shannon entropy. For example, a cluster of O cells, with O or nearly O different types of identified types may be used as an indication that the cluster is actually made up of O cells of a single, previously unknown, type for which there is no specific classifier.

In such a case, the processing apparatus 104 can disassociate, from the high-entropy cell, the generated cell type and instead classify the high-entropy cell as a novel cell type. In response, the processing apparatus can perform a number of useful action, such a i) storing information about the high-entropy cell to a data repository as a result of sensing of the biological cells; ii) transmitting a report about the high-entropy cell across a data network, and/or iii) initiating, in response to identifying the high-entropy cell, an automated process without specific user-input to initiate the automated process.

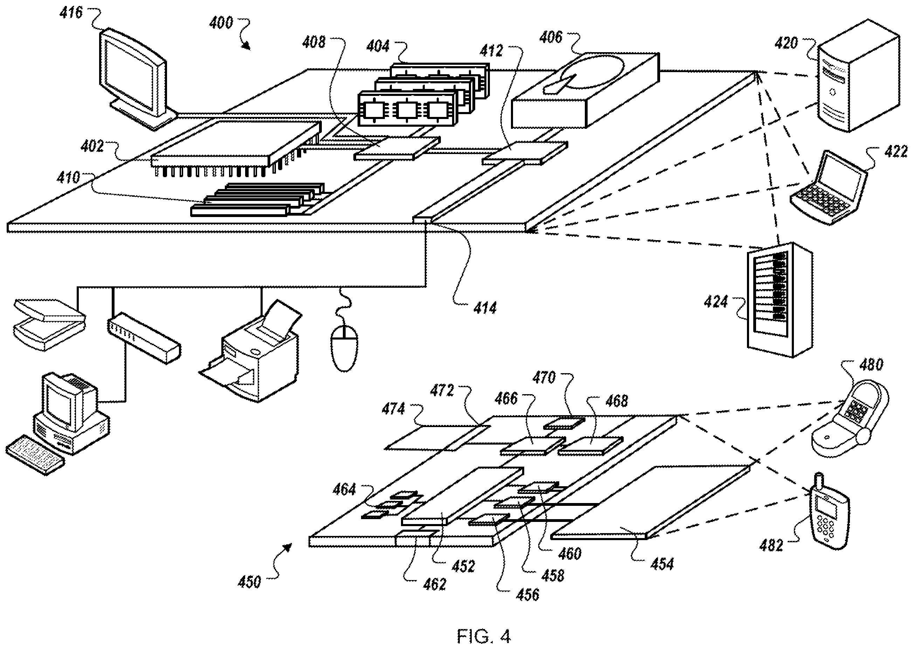

FIG. 4 shows an example of a computing device 400 and an example of a mobile computing device that can be used to implement the techniques described here. The computing device 400 is intended to represent various forms of digital computers, such as laptops, desktops, workstations, personal digital assistants, servers, blade servers, mainframes, and other appropriate computers. The mobile computing device is intended to represent various forms of mobile devices, such as personal digital assistants, cellular telephones, smart-phones, and other similar computing devices.

The computing device 400 includes a processor 402, a memory 404, a storage device 406, a high-speed interface 408 connecting to the memory 404 and multiple high-speed expansion ports 410, and a low-speed interface 412 connecting to a low-speed expansion port 414 and the storage device 406. Each of the processor 402, the memory 404, the storage device 406, the high-speed interface 408, the high-speed expansion ports 410, and the low-speed interface 412, are interconnected using various busses, and can be mounted on a common motherboard or in other manners as appropriate. The processor 402 can process instructions for execution within the computing device 400, including instructions stored in the memory 404 or on the storage device 406 to display graphical information for a GUI on an external input/output device, such as a display 416 coupled to the high-speed interface 408. In other implementations, multiple processors and/or multiple buses can be used, as appropriate, along with multiple memories and types of memory. Also, multiple computing devices can be connected, with each device providing portions of the necessary operations (e.g., as a server bank, a group of blade servers, or a multi-processor system).

The memory 404 stores information within the computing device 400. In some implementations, the memory 404 is a volatile memory unit or units. In some implementations, the memory 404 is a non-volatile memory unit or units. The memory 404 can also be another form of computer-readable medium, such as a magnetic or optical disk.

The storage device 406 is capable of providing mass storage for the computing device 400. In some implementations, the storage device 406 can be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product can also contain instructions that, when executed, perform one or more methods, such as those described above. The computer program product can also be tangibly embodied in a computer- or machine-readable medium, such as the memory 404, the storage device 406, or memory on the processor 402.

The high-speed interface 408 manages bandwidth-intensive operations for the computing device 400, while the low-speed interface 412 manages lower bandwidth-intensive operations. Such allocation of functions is exemplary only. In some implementations, the high-speed interface 408 is coupled to the memory 404, the display 416 (e.g., through a graphics processor or accelerator), and to the high-speed expansion ports 410, which can accept various expansion cards (not shown). In the implementation, the low-speed interface 412 is coupled to the storage device 406 and the low-speed expansion port 414. The low-speed expansion port 414, which can include various communication ports (e.g., USB, Bluetooth, Ethernet, wireless Ethernet) can be coupled to one or more input/output devices, such as a keyboard, a pointing device, a scanner, or a networking device such as a switch or router, e.g., through a network adapter.

The computing device 400 can be implemented in a number of different forms, as shown in the figure. For example, it can be implemented as a standard server 420, or multiple times in a group of such servers. In addition, it can be implemented in a personal computer such as a laptop computer 422. It can also be implemented as part of a rack server system 424. Alternatively, components from the computing device 400 can be combined with other components in a mobile device (not shown), such as a mobile computing device 450. Each of such devices can contain one or more of the computing device 400 and the mobile computing device 450, and an entire system can be made up of multiple computing devices communicating with each other.

The mobile computing device 450 includes a processor 452, a memory 464, an input/output device such as a display 454, a communication interface 466, and a transceiver 468, among other components. The mobile computing device 450 can also be provided with a storage device, such as a micro-drive or other device, to provide additional storage. Each of the processor 452, the memory 464, the display 454, the communication interface 466, and the transceiver 468, are interconnected using various buses, and several of the components can be mounted on a common motherboard or in other manners as appropriate.

The processor 452 can execute instructions within the mobile computing device 450, including instructions stored in the memory 464. The processor 452 can be implemented as a chipset of chips that include separate and multiple analog and digital processors. The processor 452 can provide, for example, for coordination of the other components of the mobile computing device 450, such as control of user interfaces, applications run by the mobile computing device 450, and wireless communication by the mobile computing device 450.

The processor 452 can communicate with a user through a control interface 458 and a display interface 456 coupled to the display 454. The display 454 can be, for example, a TFT (Thin-Film-Transistor Liquid Crystal Display) display or an OLED (Organic Light Emitting Diode) display, or other appropriate display technology. The display interface 456 can comprise appropriate circuitry for driving the display 454 to present graphical and other information to a user. The control interface 458 can receive commands from a user and convert them for submission to the processor 452. In addition, an external interface 462 can provide communication with the processor 452, so as to enable near area communication of the mobile computing device 450 with other devices. The external interface 462 can provide, for example, for wired communication in some implementations, or for wireless communication in other implementations, and multiple interfaces can also be used.

The memory 464 stores information within the mobile computing device 450. The memory 464 can be implemented as one or more of a computer-readable medium or media, a volatile memory unit or units, or a non-volatile memory unit or units. An expansion memory 474 can also be provided and connected to the mobile computing device 450 through an expansion interface 472, which can include, for example, a SIMM (Single In Line Memory Module) card interface. The expansion memory 474 can provide extra storage space for the mobile computing device 450, or can also store applications or other information for the mobile computing device 450. Specifically, the expansion memory 474 can include instructions to carry out or supplement the processes described above, and can include secure information also. Thus, for example, the expansion memory 474 can be provide as a security module for the mobile computing device 450, and can be programmed with instructions that permit secure use of the mobile computing device 450. In addition, secure applications can be provided via the SIMM cards, along with additional information, such as placing identifying information on the SIMM card in a non-hackable manner.

The memory can include, for example, flash memory and/or NVRAM memory (non-volatile random access memory), as discussed below. In some implementations, a computer program product is tangibly embodied in an information carrier. The computer program product contains instructions that, when executed, perform one or more methods, such as those described above. The computer program product can be a computer- or machine-readable medium, such as the memory 464, the expansion memory 474, or memory on the processor 452. In some implementations, the computer program product can be received in a propagated signal, for example, over the transceiver 468 or the external interface 462.

The mobile computing device 450 can communicate wirelessly through the communication interface 466, which can include digital signal processing circuitry where necessary. The communication interface 466 can provide for communications under various modes or protocols, such as GSM voice calls (Global System for Mobile communications), SMS (Short Message Service), EMS (Enhanced Messaging Service), or MMS messaging (Multimedia Messaging Service), CDMA (code division multiple access), TDMA (time division multiple access), PDC (Personal Digital Cellular), WCDMA (Wideband Code Division Multiple Access), CDMA2000, or GPRS (General Packet Radio Service), among others. Such communication can occur, for example, through the transceiver 468 using a radiofrequency. In addition, short-range communication can occur, such as using a Bluetooth, WiFi, or other such transceiver (not shown). In addition, a GPS (Global Positioning System) receiver module 470 can provide additional navigation- and location-related wireless data to the mobile computing device 450, which can be used as appropriate by applications running on the mobile computing device 450.

The mobile computing device 450 can also communicate audibly using an audio codec 460, which can receive spoken information from a user and convert it to usable digital information. The audio codec 460 can likewise generate audible sound for a user, such as through a speaker, e.g., in a handset of the mobile computing device 450. Such sound can include sound from voice telephone calls, can include recorded sound (e.g., voice messages, music files, etc.) and can also include sound generated by applications operating on the mobile computing device 450.

The mobile computing device 450 can be implemented in a number of different forms, as shown in the figure. For example, it can be implemented as a cellular telephone 480. It can also be implemented as part of a smart-phone 482, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms machine-readable medium and computer-readable medium refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term machine-readable signal refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing system that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network (LAN), a wide area network (WAN), and the Internet.

The computing system can include clients and servers. A client and server are generally remote from each other and typically interact through a communication network. The relationship of client and server arises by virtue of computer programs running on the respective computers and having a client-server relationship to each other.

In one example, this technology succeeds in separating immune from non-immune cells in data from three mixed tissue experiments deriving cells from kidney, synovium, and lung , which were generated with either platebased or droplet-based technologies . It also correctly rejected the nonimmune label in an example data set derived from blood . The immune and nonimmune cells exhibited significant ($p$-value $< 0.05$, Wilcoxon rank sum test) changes in gene expression for well-established immune and nonimmune cell markers, like PTPRC and CD53, indicating broadly accurate classifications of immune and nonimmune cells in peripheral tissues as well as blood.

In another example, data were generated from human blood that used Cellular Indexing of Transcriptomes and Epitopes by Sequencing (CITE-seq) to observe cell type specific protein expression. In these data, this technology identified cell types consistent with the expected protein expression: $CD19^+$ B-cells, $CD19^+CD25+$ memory B-cells, $CD19^+CD25^-CCR7^+$ naive B-cells, $CD14^{++}CD16^-$classical monocytes, $CD14^+CD16^{++}$ nonclassical monocytes, $CD3^+$ T cells, $CD45RA^+CD4^+$ naive T-cells, $CD45RO^+CD4^+$ T memory cells, $CD4^+TIGIT^+FOXP3^+$ T regulatory cells, $CD45RO^+CD8^+$ T effector memory cells, $CD56^+CD3^-$ NK cells, $CLEC10A^+$ dendritic cells (DCs), $MZB1^+$ plasma cells and $CD56^+CD3^-$ NK cells. Notably, this technology did not detect any macrophages in these data derived from blood, consistent with the idea that differentiation from monocytes occurs in tissue and not in blood.

In another example, a recent study by the Accelerating Medicines Partnership (AMP) isolated human cells ($n$ = 8,920 cells from $n$ = 26 human samples) from joint synovial tissues and performed flow cytometry in addition to scRNA-seq[15,20]. The proteins observed in this study are well-established lineage-specific markers for four distinct cell types: $CD45^+CD3^+$ T cells, $CD45^+CD3^-CD19^+$ B cells, $CD45^+CD14^+$ monocytes and $CD45^-CD31^-PDPN^+$ fibroblasts[15], allowing us to compare flow cytometry labels established previously to those generated by our approach[15]. This technology, using only the transcriptional measurements for each cell, identified 98.2% of the flow cytometry labels (95% C.I. [98.0%; 98.5%], $p$-value $< 0.001$, two-sided binomial test, $n$ = 8,334 cells;). Furthermore, This technology generated accurate classifications with as few as 200 unique genes detected per cell (95.2% average recall; 95% C.I. [76.2%; 99.9%], $p$-value $< 0.001$, two-sided binomial test; $n$ = 21 cells;), demonstrating that This technology is robust in classifying cells with low sequencing depth. Next, we turned our attention to the classification of cell types that extends beyond the flow cytometry panel to the deepest level of This technology annotations, resulting in new cell type annotations. To help validate these annotations, we note that the IMAGES identified here were consistent with well-established biology, like $FOXP3$ in T regulatory cells and $CD19$ in B cells, suggesting that This technology made accurate classifications of these cell types. However, we also note that $CD19$ transcript was detected in only 46.9% ($n$ = 734 / 1,564) of $CD45^+CD3^-CD19^+$ B cells, which demonstrates the importance of using a cell type classifier (i.e., This technology) to identify cellular phenotypes in scRNA-seq data.

The fact that this technology made accurate classifications in single cell data is surprising. Single cell data are thought to be technically distinct from sequencing experiments performed with cellular ensembles. For example, the underlying distribution of gene transcripts from single-cell data approaches Poisson (or Negative Binomial); dropouts (undetected transcripts or transcripts temporarily absent from the cell) are also a distinct feature of this data. It is hypothesized that using neural networks helped overcome this limitation due to the non-linearity of the classifier, and the ability for neural networks to make classifications based on subtle changes in gene expression profiles that distinguish cell types. This technique allowed this technology to reliably classify data from distinct samples, tissues, species and diseases sequenced with either well-based or droplet-based technologies. Changes where observed in cellular phenotypes consistent with known macrophage biology, and as such, it is possible to use this technology to study phenotypic changes of cells due to the biological context of the data set. Such consistent identification across tissues/diseases/species is not possible when using other methods (e.g., for example some surface protein measurements as in Flow Cytometry (FACS) analysis, because these are context dependent. Therefore, this is the only measurement-based, unbiased (as explained elsewhere in this document) classification that is known do this.

In another example, novel cell type populations are classified based on single-cell data. New data were introduced to compare with bootstrapped data as described above, which allowed this technology to learn from a training dataset and refine classifications for T regulatory cells, gamma delta T cells and plasmacytoid dendritic cells. Notably, pDCs were classified in an additional dataset, demonstrating that this technology learned cellular populations across distinct single cell datasets.

In another example, the technology was used to perform classification on model organisms for which flow sorted datasets are generally lacking. The technology classified cynomolgus monkey and minipig PBMCs without any additional species-specific training, by using homologous gene symbols across species.

In an example, this technology was used for the study of disease biology using four distinct datasets. This analysis revealed shared and distinct markers for cell types and identified cell types that were enriched in disease tissue. The technology identified enriched populations in the two datasets.

In an example, this technology was used on large data sets. This technology classified large (i.e., >300,000 cells) scRNA-seq data.

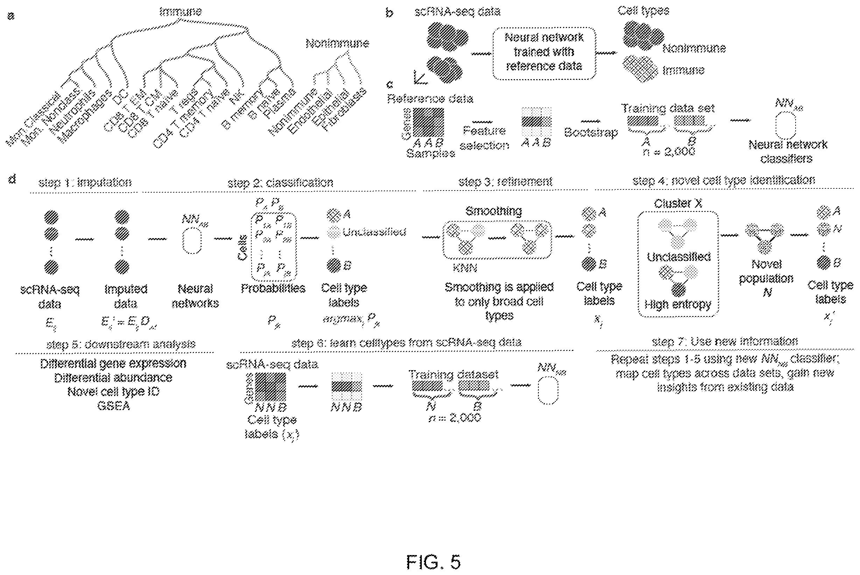

On such example is shown in FIG. 5. This technology (a) accurately and consistently maps single-cell identities to a detailed hierarchy of known immune phenotypes; (b) identifies novel cell populations and (c) surfaces disease biology from single cell data. See FIG. 5. Overall, the approach converts scRNA-seq data into an objective readout that can be used for the study of immune cells across diseases, technologies, species and tissue.

To annotate cellular phenotypes in single-cell data, technology described here can use machine learning to classify each cell in unlabeled scRNA-seq data according to a detailed hierarchy of immune phenotypes and/or non-immune phenotypes such as fibroblasts, endothelial cells, epithelial cells. It will be appreciated the other applications of this technology can be used for other phenotypes. This approach is based on neural network classifiers that were trained on a reference dataset of bulk gene expression profiles for pure cell types derived from flow-sorted cells. This training includes identifying transcriptional gene signatures for cell types using differential gene expression analysis and/or other sources of previously established gene signatures. Some of these sources can contain as few as one or two samples for each cell population, which may be too few for machine learning methods that typically require hundreds or thousands of samples. To generate useful training data, the technology described here bootstraps data sets from the rare samples in order to train machine-learning classifiers such as neural-network classifiers.

In one example implementation, this technology used a reference dataset of pure cell types, which had 713 microarray samples annotated to 157 cell types. In this dataset, ribosomal protein and mitochondrial genes were removed, samples originating from bone marrow were removed (n = 544 samples corresponding to 113 cell types remained), and a subset of genes (n = 10,808) was used that were broadly identified as exhibiting cell type specific expression previously. Within this subset, the technology identified genes that were significantly (p-value < 0.05) differentially expressed between samples annotated as different cell types in the dataset using relative count normalization. This yielded no differentially expressed genes for comparisons between memory and naive B cells, plasma cells and B cells, memory and naïve CD4 T cells, T regulatory cells and CD4 memory T cells, memory and naïve CD8 T cells, and effector memory CD8 T cells and central memory CD8 T cells; in these cases, the technology used gene signatures identified previously.

To create predictive models for cell types, first was established a training dataset from samples in the dataset by pooling samples at each level of a hierarchy shown in FIG. 5, and then bootstrapped by random resampling with replacement within each group (e.g., n = 1,000 bootstraps for immune cells, n = 1,000 bootstraps for lymphocytes, etc.), and then sampled from a random normal distribution with mean and standard deviation set by the mean and standard deviation of the resampled features. The technology we trained neural networks (n = 100) with automatically-optimized hyperparameters.

The technology then constructed k-nearest neighbor (KNN) graphs. After classification with each neural network, the label for each cell was assigned to the most frequent label of itself and of the nearest neighbors. Each cell uniqueidentifier (e.g., barcode) was assigned to the cell type label corresponding to the maximal probability derived from an average of an ensemble of neural networks (n = 100). The probabilities were then averaged over the ensemble of classifiers, and the cell type label corresponds to the maximal probability of the ensemble. The technology generated a report of the error (standard deviation) of the predictions; an individual cell barcode is labeled "Unclassified" when it has a large (2 standard deviations greater than the mean) normalized Shannon entropy within four nearest neighbors in the KNN network. This process can occur at any level of the hierarchy (e.g., unclassified T-cell subtype).

Cell barcodes labeled "Unclassified" that significantly (p < 0.01, hypergeometric test) populate a Louvain cluster in the KNN network are amended with a label corresponding to the top two expressed genes as determined by z-score transformation. Labels can carry over the last positively classified node (e.g., T-cell other).

For single cell classification, the technology's analysis started from unfiltered counts. First, removed were all cell barcodes with fewer than 200 detected genes. Next, removed were all cell barcodes with abundant (greater than the mean plus two standard deviations) percentage mitochondrial gene expression. Next, removed were all genes that were not detected in any cell barcodes, as well as all mitochondrial and ribosomal genes. Library sizes were normalized to the mean library size.

To classify cell types, the technology established a subset of the expression matrix corresponding to the intersection of gene signatures in the reference dataset and genes in the scRNA-seq matrix. After this step, each cell barcode was normalized to the mean library size, and then each gene was scaled by dividing by the maximum gene expression value in any cell barcode. Any genes with zero standard deviation were removed. Next, K-soft imputation was performed and then scaled again.

This technology identified universal and context-specific feature vectors by identifying cell type populations systematically in single cell data. We portend the use of these feature vectors for several technologies, like gene expressionbased enrichment scores/signatures" e.g. GSVA/GSEA and cell type deconvolution algorithms like CIBERSORT, which require well-established cell gene expression vectors.

To impute values for each gene for each cell, the total number of genes detected in each cell was set to the diagonal of a cell-by-cell matrix $W_{jj}$. Next, the technology established cells with direct and higher $k$-degree connections in the KNN network from the adjacency matrix $A_{jj}$ and from $k^{th}$ powers of $A_{jj}$, forming a network-based, imputation operator $D_{jj}$, which was weighted by the total number of genes detected in each cell, and normalized such that each row sums to two:

$$D_{jj} = I + \frac{\sum_k A_{jj}^k W_{jj}}{\frac{1}{2}\left(\sum_j \sum_k A_{jj}^k W_{jj}\right)}$$

The imputed expression matrix $E'_{ij}$ is then computed directly by operating on the observed expression matrix $E_{ij}$:

$$E'_{ij} = E_{ij} D_{jj}$$

**Claims**

1. A system (100) for sensing data from a sample of biological cells (106), the system comprising:

    a cell sampler (102) comprising a sample receiver and one or more sensors; wherein the cell sampler is configured to:

        sense, with the sensors, physical phenomena of biological cells in the sample receiver; and
        transmit, to processing apparatus (104), sensor-data generated from the sensing of the biological cells; and

    processing apparatus (104) comprising computer memory and one or more processors, the processing apparatus configured to:

        receive, from the cell sampler, the sensor-data;
        identify, using the sensor-data, individual cells of the biological cells;
        for each individual cell:

generate, using the sensor-data, a cell type for the individual cell, comprising submitting the sensor-data to one or more machine learning classifiers configured to receive, as input, the sensor-data and generate, as output, an indication of cell type, wherein the one or more machine-learning classifiers have been trained on an initial-corpus of training data, and wherein the cell type comprises an immune phenotype;

generate, using the sensor-data, a feature vector for the individual cell, wherein each index of the feature vector records a value to reflect a single gene expression of the individual cell;

classify, using the sensor-data, at least some of the cell types as uncommon;

generate noise that has statistical profiles that match known variations in known cells;

for each uncommon cell type:

access the feature vectors of individual cells of the uncommon cell type;

generate bootstrap vectors for the uncommon cell type by applying the generated noise to the feature vectors of individual cells of the uncommon cell type; and

generate a cell-corpus by aggregating the bootstrap vectors and the feature vectors of individual cells of the common cell type;

generate an updated-corpus of training data by incorporating at least one of the cell-corpuses to the initial-corpus; and

train one or more updated machine learning classifiers with the updated corpus.

2. The system of claim 1, wherein the processing apparatus is further configured to perform at least one of the group consisting of i) storing at least one of the cell-corpuses to a data repository as a result of sensing of the biological cells; ii) transmitting a report of at least one of the cell-corpuses across a data network, and iii) initiating, in response to generating at least one of the cell-corpuses, an automated process without specific user-input to initiate the automated process.

3. The system of claim 1 or claim 2, wherein the one or more machine-learning classifiers include a plurality of classifiers arranged in a hierarchical decision-tree that, at each of a plurality of nodes of the decision-tree having an ensemble of machine-learning classifiers that are configured to vote on a classification.

4. The system of claim 3, wherein a root node of the decision-tree has a child for immune cells and a child for non-immune cells.

5. The system of any preceding claim, wherein the processing apparatus is further configured to:

identify one of the individual cells as a high-entropy cell due to the high-entropy cell being found in a cluster with a high level of entropy;

disassociate, from the high-entropy cell, the generated cell type; and

classify the high-entropy cell as a novel cell type.

6. The system of any preceding claim, wherein the processing apparatus is further configured to:

identify one of the individual cells as a high-entropy cell due to the high-entropy cell being found in a cluster with a high level of entropy;

disassociate, from the high-entropy cell, the generated cell type; and

perform at least one of the group consisting of i) storing information about the high-entropy cell to a data repository as a result of sensing of the biological cells; ii) transmitting a report about the high-entropy cell across a data network, and iii) initiating, in response to identifying the high-entropy cell, an automated process without specific user-input to initiate the automated process.

7. The system of claim 6, wherein identifying one of the individual cells as a high-entropy cell comprises calculating a Shannon-entropy value for the high-entropy cell.

8. The system of any preceding claim, wherein the noise is generated based on statistical measures of previously-analyzed cells.

9. The system of any of claim 8, wherein the processing apparatus is further configured to generate the noise based on statistical measures of the sensor-data.

10. A method for sensing data from a sample of biological cells (106), the method comprising:

identifying (314), using sensor-data, individual cells of the biological cells;
for each individual cell:

generating (316), using the sensor-data, a cell type for the individual cell, comprising submitting the sensor-data to one or more machine learning classifiers configured to receive, as input, the sensor-data and generate, as output, an indication of cell type, wherein the one or more machine-learning classifiers have been trained on an initial-corpus of training data, and wherein the cell type comprises an immune phenotype;
generating (318), using the sensor-data, a feature vector for the individual cell, wherein each index of the feature vector records a value to reflect a single gene expression of the individual cell;
classifying (320), using the sensor-data, at least some of the cell types as uncommon;
generating noise that has statistical profiles that match known variations in known cells;

for each uncommon cell type:

accessing (322) the feature vectors of individual cells of the uncommon cell type;
generating (324) bootstrap vectors for the uncommon cell type by applying the generated noise to the feature vectors of individual cells of the uncommon cell type; and

generating (326) a cell-corpus by aggregating the bootstrap vectors and the feature vectors of individual cells of the common cell type;
generating an updated-corpus of training data by incorporating at least one of the cell-corpuses to the initial-corpus; and
training one or more updated machine learning classifiers with the updated corpus.

11. The method of claim 10, the method further comprising at least one of the group consisting of i) storing at least one of the cell-corpuses to a data repository as a result of sensing of the biological cells; ii) transmitting a report of at least one of the cell-corpuses across a data network, and iii) initiating, in response to generating at least one of the cell-corpuses, an automated process without specific user-input to initiate the automated process,

and wherein the one or more machine-learning classifiers include a plurality of classifiers arranged in a hierarchical decision-tree that, at each of a plurality of nodes of the decision-tree having an ensemble of machine-learning classifiers that are configured to vote on a classification,
and wherein a root node of the decision-tree has a child for immune cells and a child for non-immune cells.

12. The method of claim 10 or claim 11, the method further comprising:

identifying one of the individual cells as a high-entropy cell due to the high-entropy cell being found in a cluster with a high level of entropy;
disassociating, from the high-entropy cell, the generated cell type;
classifying the high-entropy cell as a novel cell type; and
performing at least one of the group consisting of i) storing information about the high-entropy cell to a data repository as a result of sensing of the biological cells; ii) transmitting a report about the high-entropy cell across a data network, and iii) initiating, in response to identifying the high-entropy cell, an automated process without specific user-input to initiate the automated process.

13. The method of claim 12, wherein identifying one of the individual cells as a high-entropy cell comprises calculating a Shannon-entropy value for the high-entropy cell.

14. The method of any of claims 10-13, wherein the noise is generated based on statistical measures of previously-analyzed cells or generating the noise based on statistical measures of the sensor-data.

15. One or more non-transitory computer storage media storing instructions that, when executed by one or more computers, cause the one or more computers to carry out the steps of the method of any of claims 10 to 14.

**Patentansprüche**

1. System (100) zum Erfassen von Daten aus einer Probe biologischer Zellen (106), wobei das System Folgendes umfasst:

   einen Zellsampler (102), der einen Probenempfänger und einen oder mehrere Sensoren umfasst; wobei der Zellsampler ausgelegt ist zum:

   Erfassen physikalischer Phänomene biologischer Zellen im Probenempfänger mit den Sensoren; und
   Übertragen, an eine Verarbeitungseinrichtung (104), von Sensordaten, die aus dem Erfassen der biologischen Zellen erzeugt werden; und
   eine Verarbeitungseinrichtung (104), die einen Computerspeicher und einen oder mehrere Prozessoren umfasst, wobei die Verarbeitungseinrichtung ausgelegt ist zum:

   Empfangen der Sensordaten von dem Zellsampler;
   Identifizieren, unter Verwendung der Sensordaten, einzelner Zellen der biologischen Zellen;
   für jede einzelne Zelle:

   Erzeugen, unter Verwendung der Sensordaten, eines Zelltyps für die einzelne Zelle, umfassend das Übermitteln der Sensordaten an einen oder mehrere Maschinenlern-Klassifizierer, die dazu ausgelegt sind, die Sensordaten als Eingabe zu empfangen und als Ausgabe eine Angabe des Zelltyps zu erzeugen, wobei der eine oder die mehreren Maschinenlern-Klassifizierer an einem Anfangskorpus von Trainingsdaten trainiert wurden und wobei der Zelltyp einen Immun-Phänotyp umfasst;
   Erzeugen, unter Verwendung der Sensordaten, eines Merkmalsvektors für die einzelne Zelle, wobei jeder Index des Merkmalsvektors einen Wert aufzeichnet, um eine Einzelgenexpression der einzelnen Zelle widerzuspiegeln; Klassifizieren, unter Verwendung der Sensordaten, mindestens einiger der Zelltypen als ungewöhnlich;
   Erzeugen von Rauschen, das statistische Profile aufweist, die mit bekannten Variationen bei bekannten Zellen übereinstimmen; für jeden ungewöhnlichen Zelltyp:

   Zugreifen auf die Merkmalsvektoren einzelner Zellen des ungewöhnlichen Zelltyps;
   Erzeugen von Bootstrap-Vektoren für den ungewöhnlichen Zelltyp durch Anwenden des erzeugten Rauschens auf die Merkmalsvektoren einzelner Zellen des ungewöhnlichen Zelltyps; und
   Erzeugen eines Zellkorpus durch Aggregieren der Bootstrap-Vektoren und der Merkmalsvektoren einzelner Zellen des gewöhnlichen Zelltyps;
   Erzeugen eines aktualisierten Korpus von Trainingsdaten durch Integrieren von mindestens einem der Zellkorpuse in den Anfangskorpus; und
   Trainieren von einem oder mehreren aktualisierten Maschinenlern-Klassifizierern mit dem aktualisierten Korpus.

2. System nach Anspruch 1, wobei die Verarbeitungseinrichtung ferner ausgelegt ist zum Durchführen von mindestens einem aus der Gruppe bestehend aus i) Speichern mindestens eines der Zellkorpuse in einem Datenrepositorium als Ergebnis des Erfassens der biologischen Zellen; ii) Übertragen eines Berichts von mindestens einem der Zellkorpuse über ein Datennetzwerk, und iii) Initiieren, als Reaktion auf das Erzeugen von mindestens einem der Zellkorpuse, eines automatisierten Prozesses ohne spezifische Benutzereingabe zum Initiieren des automatisierten Prozesses.

3. System nach Anspruch 1 oder Anspruch 2, wobei der eine oder die mehreren Maschinenlern-Klassifizierer eine Vielzahl von Klassifizierern beinhalten, die in einem hierarchischen Entscheidungsbaum angeordnet sind, der an jedem von einer Vielzahl von Knoten des Entscheidungsbaums, der ein Ensemble von Maschinenlern-Klassifizierern aufweist, die dazu ausgelegt sind, über eine Klassifizierung abzustimmen.

4. System nach Anspruch 3, wobei ein Wurzelknoten des Entscheidungsbaums einen Spross für Immunzellen und einen Spross für Nicht-Immunzellen aufweist.

5. System nach einem der vorstehenden Ansprüche, wobei die Verarbeitungseinrichtung ferner ausgelegt ist zum:

   Identifizieren einer der einzelnen Zellen als Zelle hoher Entropie, da die Zelle hoher Entropie in einem Cluster mit einem hohen Entropie-Niveau gefunden wird;
   Dissoziieren, von der Zelle hoher Entropie, des erzeugten Zelltyps; und

Klassifizieren der Zelle hoher Entropie als neuartigen Zelltyp.

6. System nach einem der vorstehenden Ansprüche, wobei die Verarbeitungseinrichtung ferner ausgelegt ist zum:

Identifizieren einer der einzelnen Zellen als Zelle hoher Entropie, da die Zelle hoher Entropie in einem Cluster mit einem hohen Entropie-Niveau gefunden wird;
Dissoziieren, von der Zelle hoher Entropie, des erzeugten Zelltyps; und
Durchführen von mindestens einem aus der Gruppe bestehend aus i) Speichern von Information über die Zelle hoher Entropie in einem Datenrepositorium als Ergebnis des Erfassens der biologischen Zellen; ii) Übertragen eines Berichts über die Zelle hoher Entropie über ein Datennetzwerk, und iii) Initiieren, als Reaktion auf das Identifizieren der Zelle hoher Entropie, eines automatisierten Prozesses ohne spezifische Benutzereingabe zum Initiieren des automatisierten Prozesses.

7. System nach Anspruch 6, wobei das Identifizieren einer der einzelnen Zellen als Zelle hoher Entropie das Berechnen eines Shannon-Entropie-Werts für die Zelle hoher Entropie umfasst.

8. System nach einem der vorhergehenden Ansprüche, wobei das Rauschen basierend auf statistischen Messungen von zuvor analysierten Zellen erzeugt wird.

9. System nach einem des Anspruchs 8, wobei die Verarbeitungseinrichtung ferner dazu ausgelegt ist, das Rauschen basierend auf statistischen Messungen der Sensordaten zu erzeugen.

10. Verfahren zum Erfassen von Daten aus einer Probe biologischer Zellen (106), wobei das Verfahren Folgendes umfasst:

Identifizieren (314), unter Verwendung von Sensordaten, einzelner Zellen der biologischen Zellen;
für jede einzelne Zelle:

Erzeugen (316), unter Verwendung der Sensordaten, eines Zelltyps für die einzelne Zelle, umfassend das Übermitteln der Sensordaten an einen oder mehrere Maschinenlern-Klassifizierer, die dazu ausgelegt sind, die Sensordaten als Eingabe zu empfangen und als Ausgabe eine Angabe des Zelltyps zu erzeugen, wobei der eine oder die mehreren Maschinenlern-Klassifizierer an einem Anfangskorpus von Trainingsdaten trainiert wurden und wobei der Zelltyp einen Immun-Phänotyp umfasst;
Erzeugen (318), unter Verwendung der Sensordaten, eines Merkmalsvektors für die einzelne Zelle, wobei jeder Index des Merkmalsvektors einen Wert aufzeichnet, um eine Einzelgenexpression der einzelnen Zelle widerzuspiegeln; Klassifizieren (320), unter Verwendung der Sensordaten, mindestens einiger der Zelltypen als ungewöhnlich;
Erzeugen von Rauschen, das statistische Profile aufweist, die mit bekannten Variationen bei bekannten Zellen übereinstimmen;
für jeden ungewöhnlichen Zelltyp:

Zugreifen (322) auf die Merkmalsvektoren einzelner Zellen des ungewöhnlichen Zelltyps;
Erzeugen (324) von Bootstrap-Vektoren für den ungewöhnlichen Zelltyp durch Anwenden des erzeugten Rauschens auf die Merkmalsvektoren einzelner Zellen des ungewöhnlichen Zelltyps; und
Erzeugen (326) eines Zellkorpus durch Aggregieren der Bootstrap-Vektoren und der Merkmalsvektoren einzelner Zellen des gewöhnlichen Zelltyps;
Erzeugen eines aktualisierten Korpus von Trainingsdaten durch Integrieren von mindestens einem der Zellkorpuse in den Anfangskorpus; und
Trainieren von einem oder mehreren aktualisierten Maschinenlern-Klassifizierern mit dem aktualisierten Korpus.

11. Verfahren nach Anspruch 10, wobei das Verfahren ferner mindestens eines aus der Gruppe umfasst, bestehend aus i) Speichern mindestens eines der Zellkorpuse in einem Datenrepositorium als Ergebnis des Erfassens der biologischen Zellen; ii) Übertragen eines Berichts von mindestens einem der Zellkorpuse über ein Datennetzwerk, und iii) Initiieren, als Reaktion auf das Erzeugen von mindestens einem der Zellkorpuse, eines automatisierten Prozesses ohne spezifische Benutzereingabe zum Initiieren des automatisierten Prozesses,

und wobei der eine oder die mehreren Maschinenlern-Klassifizierer eine Vielzahl von Klassifizierern beinhalten,

die in einem hierarchischen Entscheidungsbaum angeordnet sind, der an jedem von einer Vielzahl von Knoten des Entscheidungsbaums, der ein Ensemble von Maschinenlern-Klassifizierern aufweist, die dazu ausgelegt sind, über eine Klassifizierung abzustimmen,
und wobei ein Wurzelknoten des Entscheidungsbaums einen Spross für Immunzellen und einen Spross für Nicht-Immunzellen aufweist.

**12.** Verfahren nach Anspruch 10 oder Anspruch 11, wobei das Verfahren ferner umfasst:

Identifizieren einer der einzelnen Zellen als Zelle hoher Entropie, da die Zelle hoher Entropie in einem Cluster mit einem hohen Entropie-Niveau gefunden wird;
Dissoziieren, von der Zelle hoher Entropie, des erzeugten Zelltyps;
Klassifizieren der Zelle hoher Entropie als neuartigen Zelltyp; und
Durchführen von mindestens einem aus der Gruppe bestehend aus i) Speichern von Information über die Zelle hoher Entropie in einem Datenrepositorium als Ergebnis des Erfassens der biologischen Zellen; ii) Übertragen eines Berichts über die Zelle hoher Entropie über ein Datennetzwerk, und iii) Initiieren, als Reaktion auf das Identifizieren der Zelle hoher Entropie, eines automatisierten Prozesses ohne spezifische Benutzereingabe zum Initiieren des automatisierten Prozesses.

**13.** Verfahren nach Anspruch 12, wobei das Identifizieren einer der einzelnen Zellen als Zelle hoher Entropie das Berechnen eines Shannon-Entropie-Werts für die Zelle hoher Entropie umfasst.

**14.** Verfahren nach einem der Ansprüche 10-13, wobei das Rauschen basierend auf statistischen Messungen von zuvor analysierten Zellen erzeugt wird oder das Rauschen basierend auf statistischen Messungen der Sensordaten erzeugt wird.

**15.** Ein oder mehrere nichtflüchtige Computerspeicherungsmedien, die Anweisungen speichern, die, wenn sie von einem oder mehreren Computern ausgeführt werden, bewirken, dass der eine oder die mehreren Computer die Schritte des Verfahrens nach einem der Ansprüche 10 bis 14 ausführen.

## Revendications

**1.** Système (100) de capture de données provenant d'un échantillon de cellules biologiques (106), le système comprenant :

un échantillonneur de cellules (102) comprenant un récepteur d'échantillon et un ou plusieurs capteurs ;
l'échantillonneur de cellules étant configuré pour :

détecter, avec les capteurs, les phénomènes physiques des cellules biologiques dans le récepteur d'échantillon ; et
transmettre, à l'appareil de traitement (104), des données de capteur générées par la détection des cellules biologiques ; et
un appareil de traitement (104) comprenant une mémoire informatique et un ou plusieurs processeurs, l'appareil de traitement étant configuré pour :

recevoir, de l'échantillonneur de cellules, les données de capteur ;
identifier, à l'aide des données de capteur, des cellules individuelles parmi les cellules biologiques ;
pour chaque cellule individuelle :

générer, à l'aide des données de capteur, un type de cellule pour la cellule individuelle, comprenant la soumission des données de capteur à un ou plusieurs classificateurs d'apprentissage automatique configurés pour recevoir en entrée les données de capteur et générer en sortie une indication de type de cellule, le ou les classificateurs d'apprentissage automatique ayant été entraînés sur un corpus initial de données d'apprentissage, et le type de cellule comprenant un phénotype immunitaire ;
générer, à l'aide des données de capteur, un vecteur caractéristique pour la cellule individuelle, chaque index du vecteur caractéristique enregistrant une valeur reflétant une expression génique unique de la cellule individuelle ;

classer, à l'aide des données de capteur, au moins certains des types de cellules comme peu communs ;

générer du bruit dont les profils statistiques correspondent aux variations connues des cellules connues ;

pour chaque type de cellule peu commun :

accéder aux vecteurs caractéristiques des cellules individuelles du type de cellule peu commun ;

générer des vecteurs d'amorçage pour le type de cellule peu commun en appliquant le bruit généré aux vecteurs caractéristiques de cellules individuelles du type de cellule peu commun ; et

générer un corpus cellulaire en agrégeant les vecteurs d'amorçage et les vecteurs caractéristiques de cellules individuelles du type de cellule commun ;

générer un corpus mis à jour de données d'entraînement en incorporant au moins un des corpus cellulaires au corpus initial ; et

entraîner un ou plusieurs classificateurs d'apprentissage automatique mis à jour avec le corpus mis à jour.

2. Système selon la revendication 1, l'appareil de traitement étant en outre configuré pour réaliser au moins une action du groupe comprenant i) le stockage d'au moins un des corpus cellulaires dans un dépôt de données à la suite de la détection des cellules biologiques ; ii) la transmission d'un rapport d'au moins un des corpus cellulaires à un réseau de données, et iii) l'initiation, en réponse à la génération d'au moins un des corpus cellulaires, d'un processus automatisé sans entrée utilisateur spécifique pour initier le processus automatisé.

3. Système selon la revendication 1 ou la revendication 2, le ou les classificateurs d'apprentissage automatique comprenant une pluralité de classificateurs agencés dans un arbre de décision hiérarchique qui, au niveau de chacun d'une pluralité de nœuds de l'arbre de décision présentant un ensemble de classificateurs d'apprentissage automatique qui sont configurés pour voter sur une classification.

4. Système selon la revendication 3, un nœud racine de l'arbre de décision ayant un enfant pour les cellules immunitaires et un enfant pour les cellules non immunitaires.

5. Système selon l'une quelconque des revendications précédentes, l'appareil de traitement étant configuré en outre pour :

identifier l'une des cellules individuelles comme une cellule à entropie élevée en raison de la cellule à entropie élevée trouvée dans un amas avec un niveau élevé d'entropie ;

dissocier, de la cellule à entropie élevée, le type de cellule généré ; et

classer la cellule à entropie élevée comme un nouveau type de cellule.

6. Système selon l'une quelconque des revendications précédentes, l'appareil de traitement étant configuré en outre pour :

identifier l'une des cellules individuelles comme une cellule à entropie élevée en raison de la cellule à entropie élevée trouvée dans un amas avec un niveau élevé d'entropie ;

dissocier, de la cellule à entropie élevée, le type de cellule généré ; et

réaliser au moins une action du groupe comprenant i) le stockage d'informations sur la cellule à entropie élevée dans un dépôt de données à la suite de la détection des cellules biologiques ; ii) la transmission d'un rapport sur la cellule à entropie élevée à un réseau de données ;

et iii) l'initiation, en réponse à l'identification de la cellule à entropie élevée, d'un processus automatisé sans entrée utilisateur spécifique pour initier le processus automatisé.

7. Système selon la revendication 6, l'identification de l'une des cellules individuelles comme une cellule à entropie élevée comprenant le calcul d'une valeur d'entropie de Shannon pour la cellule à entropie élevée.

8. Système selon l'une quelconque des revendications précédentes, le bruit étant généré sur la base de mesures statistiques de cellules analysées précédemment.

9. Système selon l'une de la revendication 8, l'appareil de traitement étant en outre configuré pour générer le bruit sur la base de mesures statistiques des données de capteur.

10. Procédé de détection de données provenant d'un échantillon de cellules biologiques (106), le procédé comprenant :

l'identification (314), à l'aide de données de capteurs, de cellules individuelles parmi les cellules biologiques ; pour chaque cellule individuelle :

la génération (316), à l'aide des données de capteur, d'un type de cellule pour la cellule individuelle, comprenant la soumission des données de capteur à un ou plusieurs classificateurs d'apprentissage automatique configurés pour recevoir en entrée les données de capteur et générer en sortie une indication de type de cellule, le ou les classificateurs d'apprentissage automatique ayant été entraînés sur un corpus initial de données d'apprentissage, et le type de cellule comprenant un phénotype immunitaire ;
la génération (318), à l'aide des données de capteur, d'un vecteur caractéristique pour la cellule individuelle, chaque index du vecteur caractéristique enregistrant une valeur reflétant une expression génique unique de la cellule individuelle ;
le classement (320), à l'aide des données de capteur, d'au moins certains des types de cellules comme peu communs ;
la génération de bruit dont les profils statistiques correspondent aux variations connues des cellules connues ;
pour chaque type de cellule peu commun :

l'accès (322) aux vecteurs caractéristiques des cellules individuelles du type de cellule peu commun ;
la génération (324) de vecteurs d'amorçage pour le type de cellule peu commun en appliquant le bruit généré aux vecteurs caractéristiques de cellules individuelles du type de cellule peu commun ; et
la génération (326) d'un corpus cellulaire en agrégeant les vecteurs d'amorçage et les vecteurs caractéristiques de cellules individuelles du type de cellule commun ;
la génération d'un corpus mis à jour de données d'entraînement en incorporant au moins un des corpus cellulaires au corpus initial ; et
l'entraînement d'un ou plusieurs classificateurs d'apprentissage automatique mis à jour avec le corpus mis à jour.

11. Procédé selon la revendication 10, le procédé comprenant en outre au moins une action du groupe comprenant i) le stockage d'au moins un des corpus cellulaires dans un dépôt de données à la suite de la détection des cellules biologiques ; ii) la transmission d'un rapport d'au moins un des corpus cellulaires à un réseau de données ; et iii) l'initiation, en réponse à la génération d'au moins un des corpus cellulaires, d'un processus automatisé sans entrée utilisateur spécifique pour initier le processus automatisé,

et le ou les classificateurs d'apprentissage automatique incluant une pluralité de classificateurs agencés en un arbre de décision hiérarchique qui, au niveau de chacun d'une pluralité de nœuds de l'arbre de décision présentant un ensemble de classificateurs d'apprentissage automatique qui sont configurés pour voter sur une classification,
et un nœud racine de l'arbre de décision ayant un enfant pour les cellules immunitaires et un enfant pour les cellules non immunitaires.

12. Procédé selon la revendication 10 ou la revendication 11, le procédé comprenant en outre :

l'identification d'une des cellules individuelles comme une cellule à entropie élevée en raison de la cellule à entropie élevée retrouvée dans un amas avec un niveau élevé d'entropie ;
la dissociation, de la cellule à entropie élevée, du type de cellule généré ;
le classement de la cellule à entropie élevée comme un nouveau type de cellule ; et
la réalisation d'au moins une action du groupe comprenant i) le stockage d'informations sur la cellule à entropie élevée dans un dépôt de données à la suite de la détection des cellules biologiques ; ii) la transmission d'un rapport sur la cellule à entropie élevée sur un réseau de données ; et iii) l'initiation, en réponse à l'identification de la cellule à entropie élevée, d'un processus automatisé sans entrée utilisateur spécifique pour initier le processus automatisé.

13. Procédé selon la revendication 12, l'identification de l'une des cellules individuelles comme une cellule à entropie élevée comprenant le calcul d'une valeur d'entropie de Shannon pour la cellule à entropie élevée.

14. Procédé selon l'une quelconque des revendications 10 à 13, le bruit étant généré sur la base de mesures statistiques de cellules analysées précédemment ou de génération du bruit sur la base de mesures statistiques des données de capteur.

15. Un ou plusieurs supports de stockage informatiques non transitoires stockant des instructions qui, lorsqu'elles sont exécutées par un ou plusieurs ordinateurs, amènent le ou les ordinateurs à mettre en œuvre les étapes du procédé selon l'une quelconque des revendications 10 à 14.

FIG. 1

FIG. 2

FIG. 3

FIG. 4

FIG. 5